# EUROPEAN PATENT SPECIFICATION

(11) **EP 1 397 994 B1**
(45) Date of publication and mention of the grant of the patent: **22.02.2006**
(21) Application number: 03255560.9
(22) Date of filing: 05.09.2003
(51) Int. Cl.: A61B 5/08, A61B 5/087

(54) **Apparatus for measuring the strength of a person's respiratory muscles**
Vorrichtung zur Ermittlung der Atmungsmuskulären Kraft einer Person
Appareil pour mesurer la force des muscles respiratoires d'une personne

(30) Priority: 11.09.2002 GB 0221044
(43) Date of publication of application: 17.03.2004
(73) Proprietor: MICRO MEDICAL LIMITED, Kent, MEI 2AZ (GB)
(72) Inventor: Lawson, Christopher Patrick, Rochester Kent ME1 2BN (GB)
(74) Representative: Jones, Graham Henry

(56) References cited:
- WO-A-98/14115
- US-A- 3 924 612
- US-A- 4 966 141
- US-A- 6 066 101
- US-A- 6 067 983
- US-B1- 6 379 311

## Description

This invention relates to apparatus for measuring the strength of a person's respiratory muscles.

Apparatus for measuring the strength of a person's respiratory muscles is known. The known apparatus comprises a mouthpiece and a person is required to inhale against an obstruction in the apparatus in order to see what respiratory pressure the person can generate. This respiratory pressure is known as the maximum inspiratory pressure. The measurement of the maximum inspiratory pressure simply by inhaling against an obstruction does not give an in-depth understanding of the person's respiratory muscles. Such an in-depth understanding of the person's respiratory muscles would be advantageous in many situations. For example, with athletes, an in-depth understanding of an athlete's respiratory muscles could lead to a finding that the athlete's respiratory muscles were weak over a certain respiratory pressure range, and were strong over a different respiratory pressure range. In this case, the athlete could then train his or her lungs especially over the weak respiratory pressure range, in order to strengthen the weak respiratory muscles. Also by way of example, it is mentioned that the majority of those people who die from major surgery actually die from respiratory failure. This is especially so if the major surgery is heart surgery. If an in-depth understanding of the person's respiratory muscles could be obtained before major surgery, then any weakness in the person's respiratory muscles could be identified. The person could then be given pre-operative exercises in order to strengthen their respiratory muscles over the weak respiratory pressure range or pressure ranges. This would then increase the person's chances of survival after major surgery because it would reduce the likelihood of that person dying from respiratory failure after the major surgery.

WO 98/14115 discloses apparatus for measuring the strength of a person's respiratory muscles. The apparatus comprises a mouthpiece, a flow transducer, a pressure transducer, a variable orifice valve, a motor for operating the variable orifice valve, and microprocessor control means.

It is an aim of the present invention to provide apparatus which enables an in-depth understanding of a person's respiratory muscles to be obtained.

Accordingly, the present invention provides apparatus for measuring the strength of a person's respiratory muscles, which apparatus comprises a mouthpiece for the person, a flow transducer, a pressure transducer, a variable orifice valve, a motor for operating the variable orifice valve, and microprocessor control means, the microprocessor control means being such that it is able to control the motor to cause the variable orifice valve to vary its orifice size and thereby to maintain a constant predetermined pressure and enable the measurement of the flow rate generated by the person, or to maintain a constant predetermined flow rate and enable the measurement of the pressure generated by the person, characterized in that the variable orifice valve is a rotary variable orifice valve comprising a cylindrical member, a longitudinally extending bore in the cylindrical member, a lateral aperture positioned in a wall of the cylindrical member and between ends the cylindrical member, a sleeve, a longitudinally extending bore in the sleeve, and a lateral aperture positioned in a wall of the sleeve between ends of the sleeve.

Usually, the flow rate or the pressure generated by the person will be generated by inhalation, but exhalation may be employed if desired.

Preferably, the apparatus of the invention will be used such that the microprocessor control means maintains different constant predetermined pressures, and measures the flow rate generated by the person at these constant predetermined pressures. If desired however, the apparatus of the present invention may be used such that the microprocessor control means maintains different predetermined flow rates and measures the pressure generated by the person. Either way, a maximum inspiratory pressure curve can be built up, and weak parts of the person's respiratory muscles can be seen from the curve. Corrective respiratory exercises can then be prescribed to strengthen any weak range or ranges of the respiratory muscles. For persons with weak respiratory muscles, the variable orifice will generally be small for the maximum inspired flow rate at a chosen pressure. For persons with strong respiratory muscles, the variable orifice will generally be large for the maximum inspired flow rate at a chosen pressure. Various exercises can be prescribed for persons with weak respiratory muscles over various ranges in order to improve the strength of the respiratory muscles over these ranges.

The apparatus may include a control circuit, the flow transducer being connected to the control circuit, the pressure transducer being connected to the variable orifice valve and to the control circuit, and the control circuit being connected to the microprocessor control means.

The microprocessor control means may comprise a microprocessor circuit, display means, and a keypad.

The display means may be a display screen and/or a hard copy print device.

Preferably, the mouthpiece has a flange at the end of the mouthpiece that goes into the person's mouth. The flange helps the person's mouth to seal around the mouthpiece during the inhalation.

With the rotary variable orifice valve, friction may be independent of applied pressure. The relationship between the resistance to flow and rotation of the valve is able easily to be adjusted by the shape of the orifice.

The rotary variable orifice valve may have an orifice which is of a shape that causes the resistance to flow of the rotary variable orifice valve to increase with rotation. Preferably, the orifice in the rotary variable orifice valve is of a triangular shape. Other shapes may be employed if desired.

The rotary variable orifice valve is preferably such that the sleeve is a rotational fit with respect to the cylindrical member. The sleeve will normally be a rotational fit over the cylindrical member. Other types of rotary variable orifice valve may be employed so that, for example, the rotary variable orifice valve may be one in which the cylindrical member rotates and the sleeve remains stationary.

The rotary variable orifice valve may be one in which the cylindrical member has an aperture, the sleeve has the orifice, and the aperture and the orifice are positioned such that they overlap as the sleeve rotates. Alternatively, the rotary variable orifice valve may be one in which the cylindrical member has the orifice, the sleeve has an aperture, and the aperture and the orifice are positioned such that they overlap as the sleeve rotates. Alternatively, the rotary variable orifice valve may be one in which the orifice is positioned partly in the cylindrical member and partly in the sleeve.

The use of the rotary variable orifice valve is better than a flat plate variable orifice valve. A flat plate variable orifice valve may cause friction which is dependent upon the amount of pressure being placed upon the plate. This friction may need to be overcome by the use of a motor which is larger than the motor required for a rotary variable orifice valve which does not suffer from generated friction. The use of a smaller motor may in turn enable the apparatus of the present invention to be produced in a smaller size. Alternatively or in addition, any battery power employed to drive the motor may be less for a rotary variable orifice valve than for a flat plate orifice valve.

Embodiments of the invention will now be described solely by way of example and with reference to the accompanying drawings in which:
Figure 1 shows first apparatus of the present invention;
Figure 2 is a block circuit diagram of the apparatus shown in Figure 1;
Figure 3 shows a curve obtained by measuring pressure against flow and obtaining maximum inspired flow rates for different pressures; and
Figure 4 is a perspective view of a rotary variable orifice valve which is able to be used in the apparatus shown in Figure 1.

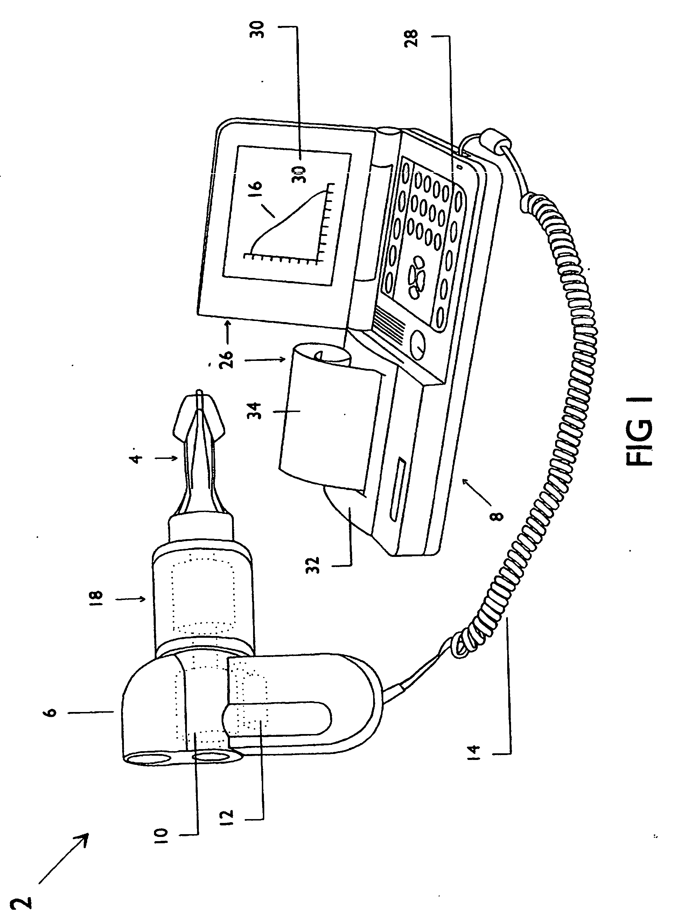

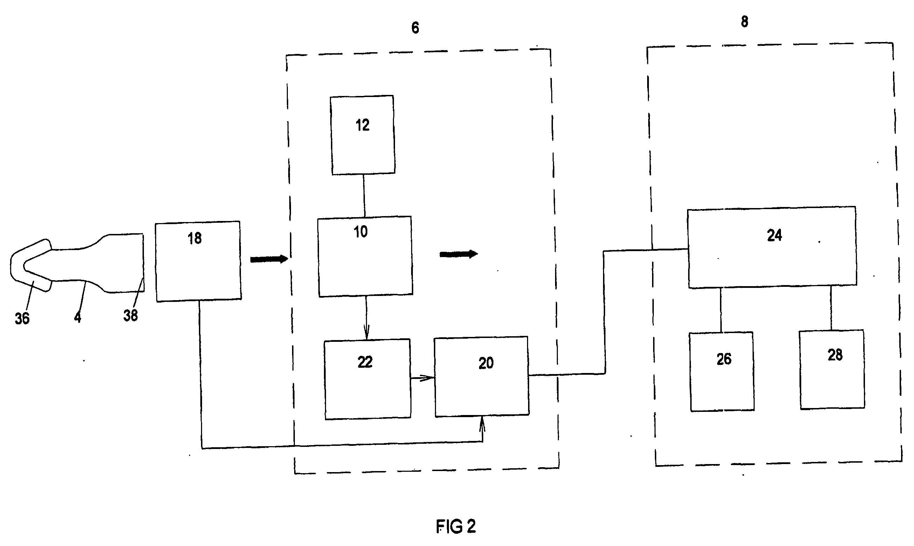

Referring to Figures 1 and 2, there is shown apparatus 2 for measuring the strength of a person's respiratory muscles. The apparatus 2 comprises a mouthpiece 4 for being inhaled through on by the person, a variable orifice valve arrangement 6, and microprocessor control means 8. The variable orifice valve arrangement 6 comprises a variable orifice valve 10 and a motor 12 for operating the variable orifice valve 10. The microprocessor control means 8 is such that it is able to control the motor 12 to cause the variable orifice valve 10 to vary its orifice size and thereby to maintain a constant predetermined pressure and enable the measurement of the flow rate generated by the person, or to maintain a constant predetermined flow rate and enable the measurement of the pressure generated by the person.

The variable orifice valve arrangement 6 also comprises a control circuit 20 and a pressure transducer 22. A flow transducer 18 is positioned between the mouthpiece 4 and the variable orifice valve arrangement 6.

The constant respiratory pressure transducer 6 is connected to the microprocessor control means 8 by a lead 14 as shown in Figure 1.

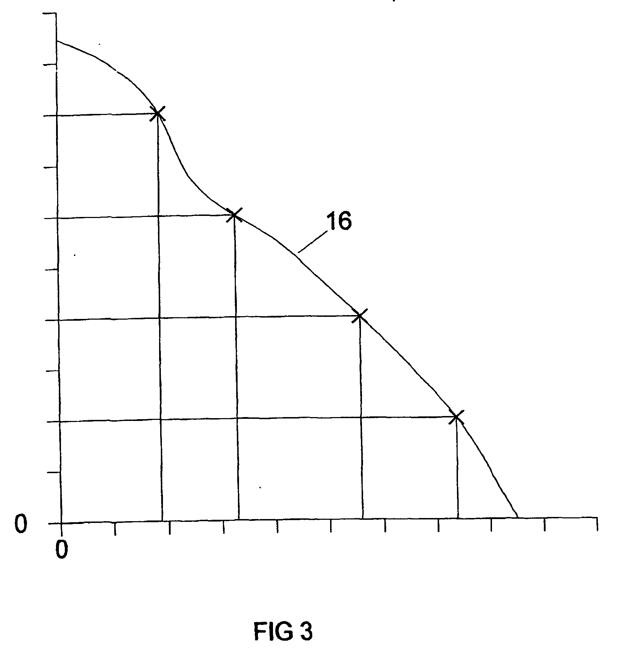

During use of the apparatus 2, for a person with weak lungs, the orifice in the variable orifice valve 10 will usually be relatively small for the maximum inspired flow rate. For a person with strong lungs, the orifice in the variable orifice valve 10 will usually be relatively large for the maximum inspired flow rate. Measurements can be taken of pressure against flow in order to build up a maximum inspiratory pressure curve 16 as shown in Figure 3. If the measurements being taken fluctuate due to uneven inhalation by the person, then a suitable algorithm may be employed to provide an average for each measurement. The curve 16 is then useful for identifying areas of weakness in the person's respiratory muscles. The person, for example a patient shortly to undergo major heart surgery, or an athlete, can then be given remedial exercises to strengthen their respiratory muscles over the weak range or ranges. In the case of persons about to undergo major surgery, the improved respiratory muscles will increase their chances of survival. In the case of athletes, improved respiratory muscles may result in improved performances.

As shown in Figure 2, the flow transducer 18 is connected to the control circuit 20. The pressure transducer 22 is connected to the variable orifice valve 10 and to the control circuit 20. The control circuit 20 is connected to a microprocessor circuit 24 of the microprocessor control means 8. The microprocessor circuit 24 is also connected to display means 26 and a keypad 28.

As shown in Figure 1, the display means 26 comprises a display screen 30 and a hard copy print device 32. The display screen 30 is shown displaying a maximum inspiratory pressure curve 16. The print device 32 is shown having provided a hard copy print 34.

The mouthpiece 4 has a flange 36 at the end of the mouthpiece that goes into the person's mouth. The flange 36 helps the person's mouth to seal around the mouthpiece 4 during inhalation. The other end 38 of the mouthpiece 4 is cylindrical for being a push fit over a cylindrical part of the constant respiratory pressure transducer 6.

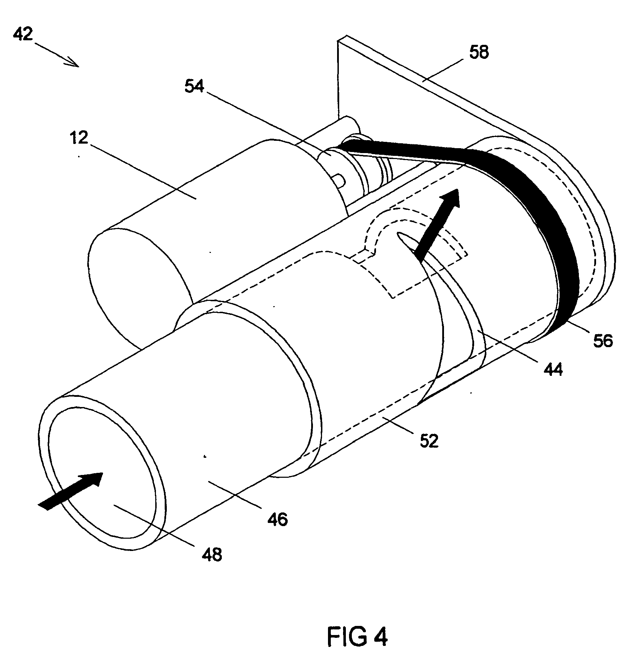

Figure 4 shows a rotary variable orifice valve 42 which is a preferred form of the variable orifice valve 10 shown in Figure 1. The rotary variable orifice valve 42 does not generate friction so that friction is independent of applied pressure. The relationship between resistance to flow and rotation of the valve is easily adjusted by adjusting the shape of an orifice 44. The orifice 44 is of a shape that causes the resistance to flow of the rotary variable orifice valve 42 to increase with rotation. More specifically, the orifice 44 is of a triangular shape as shown.

The rotary variable orifice valve 42 comprises a cylindrical member 46 having a bore 48 and a rectangular aperture 50. The orifice 44 is in a sleeve 52 which is a rotational fit over the cylindrical member 46. As shown in Figure 4, the cylindrical member 46 is in the form of a short tube. During use of the rotary variable orifice valve 42, the sleeve 52 rotates over the cylindrical member 46, and the orifice 44 overlaps by varying amounts the rectangular aperture 50. In this way, the effective size of the orifice 44 is varied. Air flow along the bore 48 and through the orifice 44 is shown by arrows.

The rotation of the sleeve 52 is controlled by the motor 12. The motor 12 is mounted on one side of the sleeve 52. The motor 12 has a pulley 54 which drives an endless drive belt 56. The drive belt 56 is in frictional engagement with the outside of the sleeve 52 as shown. Thus rotation of the pulley 54 clockwise or anticlockwise, causes a corresponding rotation of the sleeve 52 via the drive belt 56. The motor 12 is mounted on a motor mounting plate 58.

It is to be appreciated that the embodiments of the invention described above with reference to the accompanying drawings have been given by way of example only and that modifications may be effected. For example, with reference to Figure 4, the motor 12 may be arranged to drive the sleeve 56 by means other than the drive belt 56. Thus, for example, the drive could be via toothed wheels. Also, if desired, the motor 12 could be mounted in line with the cylindrical member 46 and then connected to the sleeve 52 by an appropriate drive arrangement. Alternatively, or in addition, the motor may be mains operated. In Figure 3, the curve 16 has been obtained by causing the microprocessor control means 8 to control the motor 12 to cause the variable orifice valve 10 to vary its orifice size and thereby to maintain constant predetermined pressures, so that the flow rate generated by the person can be measured. If desired however, the curve 16 may be obtained by causing the microprocessor control means 16 to control the motor 12 to cause the variable orifice valve 10 to vary its orifice size and thereby to maintain constant predetermined flow rates, so that the pressure generated by the person can be measured.

## Claims

1. Apparatus (2) for measuring the strength of a person's respiratory muscles, which apparatus (2) comprises a mouthpiece (4) for the person, a flow transducer (18), a pressure transducer (6), a variable orifice valve (10), a motor (12) for operating the variable orifice valve (10), and microprocessor control means (8), the microprocessor control means (8) being such that it is able to control the motor (12) to cause the variable orifice valve (10) to vary its orifice size and thereby to maintain a constant predetermined pressure and enable measurement of the flow rate generated by the person, or to maintain a constant predetermined flow rate and enable the measurement of the pressure generated by the person, **characterized in that** the variable orifice valve (10) is a rotary variable orifice valve (10) comprising a cylindrical member (46), a longitudinally extending bore (48) in the cylindrical member (46), a lateral aperture (50) positioned in a wall of the cylindrical member (46) and between ends of the cylindrical member (46), a sleeve (52), a longitudinally extending bore in the sleeve (52), and a lateral aperture (44) positioned in a wall of the sleeve (52) between ends of the sleeve (52).

2. Apparatus (2) according to claim 1 and including a control circuit (20), the flow transducer (18) being connected to the control circuit (20), the pressure transducer (6) being connected to the variable orifice valve (10) and to the control circuit (20), and the control circuit (20) being connected to the microprocessor control means (8).

3. Apparatus (2) according to claim 1 or claim 2 in which the microprocessor control means (8) comprises a microprocessor circuit (24), display means (26), and a keypad (28).

4. Apparatus (2) according to claim 3 in which the display means (26) is a display screen (30) and/or a hard copy print device (32).

5. Apparatus (2) according to any one of the preceding claims in which the mouthpiece (4) has a flange (36) at the end of the mouthpiece (4) that goes into the person's mouth.

6. Apparatus (2) according to any one of the preceding claims in which the rotary variable orifice valve (42) has an orifice (44) which is of a shape that causes the resistance to flow of the rotary variable orifice valve (42) to increase with rotation.

7. Apparatus (2) according to claim 6 in which the orifice (44) in the rotary variable orifice valve (42) is of a triangular shape.

8. Apparatus (2) according to any one of the preceding claims in which the sleeve (52) is a rotational fit over the cylindrical member (46).

9. Apparatus (2) according to any one of the preceding claims in which the cylindrical member (46) has an aperture (50), the sleeve (52) has the orifice (44), and the aperture (50) and the orifice (44) are positioned such that they overlap as the sleeve (52) rotates.

10. Apparatus (2) according to any one of claims 1 - 8 in which the cylindrical member (46) has the orifice (44), the sleeve (52) has an aperture 50), and the aperture and the orifice (44) are positioned such that they overlap as the sleeve (52) rotates.

11. Apparatus according to any one of claims 1 - 8 in which the orifice (44) is positioned partly in the cylindrical member (46) and partly in the sleeve (52).

## Patentansprüche

1. Vorrichtung (2) zur Messung der Stärke der Atemmuskulatur einer Person, wobei die Vorrichtung (2) ein Mundstück (4) für die Person, einen Strömungswandler (18), einen Druckwandler (6), ein Ventil (10) mit variabler Drossel, einen Motor (12) zur Betätigung des Ventils (10) mit variabler Drossel und ein Mikroprozessorsteuerungsmittel (8) aufweist, wobei das Mikroprozessorsteuerungsmittel (8) so eingerichtet ist, dass es den Motor (12) so steuern kann, dass das Ventil (10) mit variabler Drossel veranlasst wird, seine Drosselgröße zu ändern, um **dadurch** einen konstanten vorgegebenen Druck aufrechtzuerhalten und die Messung des Durchsatzes, der von der Person erzeugt wird, zu ermöglichen, oder um einen vorgegebenen konstanten Durchsatz aufrechtzuerhalten und die Messung des von der Person erzeugten Drucks zu ermöglichen, **dadurch gekennzeichnet, dass** das Ventil (10) mit variabler Drossel ein Drehschieber (10) mit variabler Drossel ist, der ein zylindrisches Element (46), eine sich in Längsrichtung erstreckende Bohrung (48) im zylindrischen Element (46), eine seitliche Öffnung (50), die in der Wand des zylindrischen Elements (46) und zwischen den Enden des zylindrischen Elements (46) positioniert ist, eine Hülse (52), eine sich in Längsrichtung erstreckende Bohrung in der Hülse (52) und eine seitliche Öffnung (44), die in der Wand der Hülse (52) und zwischen den Enden der Hülse (52) positioniert ist, aufweist.

2. Vorrichtung (2) nach Anspruch 1, die auch eine Steuerschaltung (20) enthält, wobei der Strömungswandler (18) mit der Steuerschaltung (20), der Druckwandler (6) mit dem Ventil (10) mit variabler Drossel und mit der Steuerschaltung (20) und die Steuerschaltung (20) mit dem Mikroprozessorsteuerungsmittel (8) verbunden ist.

3. Vorrichtung (2) nach Anspruch 1 oder Anspruch 2, bei der das Mikroprozessorsteuerungsmittel (8) eine Mikroprozessorschaltung (24), ein Anzeigemittel (26) und eine Tastatur (28) aufweist.

4. Vorrichtung (2) nach Anspruch 3, bei der das Anzeigemittel (26) ein Anzeigebildschirm (30) und/oder ein Druckgerät (32) für Hardcopys ist.

5. Vorrichtung (2) nach einem der vorigen Ansprüche, bei der das Mundstück (4) einen Flansch (36) am Ende des Mundstücks (4) hat, den die Person in den Mund nimmt.

6. Vorrichtung (2) nach einem der vorigen Ansprüche, bei der der Drehschieber (42) mit variabler Drossel eine Drossel (44) mit einer solchen Form hat, die bewirkt, dass der Strömungswiderstand des Drehschiebers (42) mit variabler Drossel mit der Drehung zunimmt .

7. Vorrichtung (2) nach Anspruch 6, bei der die Drossel (44) im Drehschieber (42) mit variabler Drossel eine Dreieckform hat.

8. Vorrichtung (2) nach einem der vorigen Ansprüche, bei der die Hülse (52) drehbar über dem zylindrischen Element (46) sitzt.

9. Vorrichtung (2) nach einem der vorigen Ansprüche, bei der das zylindrische Element (46) eine Drossel (50), die Hülse (52) die Drossel (44) enthält, und die Öffnung (50) sowie die Drossel (44) so positioniert sind, dass sie einander überlappen, wenn sich die Hülse (52) dreht.

10. Vorrichtung (2) nach einem der Ansprüche 1 bis 8, bei der das zylindrische Element (46) die Drossel (44) enthält, die Hülse (52) eine Öffnung (50) hat, und die Öffnung sowie die Drossel (44) so positioniert sind, dass sie einander überlappen, wenn sich die Hülse (52) dreht.

11. Vorrichtung nach einem der Ansprüche 1 bis 8, bei der die Drossel (44) teilweise im zylindrischen Element (46) und teilweise in der Hülse (52) positioniert ist.

## Revendications

1. Appareil (2) pour mesurer la force des muscles respiratoires d'une personne, ledit appareil (2) comprenant une embouchure (4) pour la personne, un transducteur de débit (18), un transducteur de pression (6), une soupape (10) à orifice variable, un moteur (12) pour actionner la soupape (10) à orifice variable et des moyens de commande (8) à microprocesseur, les moyens de commande (8) à microprocesseur servant à commander le moteur (12) pour qu'il modifie la taille de l'orifice de la soupape (10) à orifice variable, ce qui permet de maintenir une pression prédéterminée et constante et de mesurer le débit d'écoulement créé par la personne ou de maintenir un débit d'écoulement prédéterminé et constant, rendant ainsi possible la mesure de la pression créée par la personne, **caractérisé en ce que** la soupape (10) à orifice variable comprend un organe cylindrique (46), un alésage (48) qui s'étend dans le sens de la longueur à l'intérieur de l'organe cylindrique (46), une ouverture latérale (50) ménagée dans la paroi de l'organe cylindrique (46) et entre les extrémités de l'organe cylindrique (46), un manchon (52), un alésage qui s'étend dans le sens de la longueur dans le manchon (52) et une ouverture latérale (44) ménagée dans la paroi du manchon (52) entre les extrémités du manchon (52).

2. Appareil (2) selon la revendication 1, qui comprend un circuit de commande (20), le transducteur d'écoulement (18) étant relié au circuit de commande (20), le transducteur de pression (6) étant relié à la soupape (10) à orifice variable et au circuit de commande (20), le circuit de commande (20) étant relié aux moyens de commande (8) à microprocesseur.

3. Appareil (2) selon les revendications 1 ou 2, dans lequel les moyens de commande (8) à microprocesseur sont constitués d'un circuit (24) pour microprocesseur, de moyens d'affichage (26) et d'un clavier numérique (28).

4. Appareil (2) selon la revendication 3 dans lequel les moyens d'affichage (26) consistent en un écran d'affichage (30) et/ou en un dispositif (32) d'impression sur papier.

5. Appareil (2) selon l'une quelconque des précédentes revendications, dans lequel l'embouchure (4) présente à l'extrémité de l'embouchure (4) une collerette (36) qui pénètre dans la bouche de la personne.

6. Appareil (2) selon l'une quelconque des précédentes revendications, dans lequel la soupape rotative (42) à orifice variable a un orifice (44) dont la forme induit dans la soupape (42)à orifice variable une résistance à l'écoulement qui augmente avec sa rotation.

7. Appareil (2) selon la revendication 6, dans lequel fotifice (44) de la soupape rotative (42) à orifice variable a une forme triangulaire.

8. Appareil (2) selon l'une quelconque des précédentes revendications, dans lequel le manchon (52) est empêché de tourner sur l'organe cylindrique (46).

9. Appareil (2) selon l'une quelconque des précédentes revendications, dans lequel l'organe cylindrique (46) présente une ouverture (50), le manchon (52) un orifice (44), l'ouverture (50) et l'orifice (44) étant situés de façon à se chevaucher lorsque le manchon (52) tourne.

10. Appareil (2) selon l'une quelconque des revendications 1 à 8, dans lequel l'organe cylindrique (46) présente l'orifice (44) et le manchon (52) une ouverture (50), l'ouverture et l'orifice (44) étant situés de façon à se chevaucher lorsque le manchon (52) tourne.

11. Appareil selon l'une quelconque des revendications 1 à 8, dans lequel l'orifice (44) est situé en partie sur l'organe cylindrique (46) et en partie sur le manchon (52).
